(19) Europäisches Patentamt
European Patent Office
Office européen des brevets

(11) Publication number: **0 223 681 B1**

# EUROPEAN PATENT SPECIFICATION

(45) Date of publication of patent specification: **22.05.91**

(51) Int. Cl.⁵: **A01N 25/24**, A61K 47/00

(21) Application number: **86402413.8**

(22) Date of filing: **28.10.86**

The file contains technical information submitted after the application was filed and not included in this specification

(54) **Alcohol-based antimicrobial compositions and method for enhancing their efficacity.**

(30) Priority: **28.10.85 US 791668**

(43) Date of publication of application:
**27.05.87 Bulletin 87/22**

(45) Publication of the grant of the patent:
**22.05.91 Bulletin 91/21**

(84) Designated Contracting States:
**AT BE CH DE FR GB IT LI LU NL SE**

(56) References cited:
**EP-A- 0 030 576       FR-A- 2 191 941
GB-A- 1 023 895       GB-A- 1 465 665
GB-A- 2 017 491       US-A- 4 434 181**

(73) Proprietor: **CALGON CORPORATION
Route 60-Campbell's Run Road
Robinson Township Pennsylvania 15205(US)**

(72) Inventor: **White, John H.
3939 Flora Place
Saint Louis Missouri 63110(US)**

(74) Representative: **Ahner, Francis et al
CABINET REGIMBEAU, 26, avenue Kléber
F-75116 Paris(FR)**

## Description

Alcohol-based skin disinfectants, because of their high vapor pressure, evaporate quickly. Thus, when they are applied to skin, alcohol concentration and contact time with bacteria and other microorganisms are minimized due to evaporative loss.

Such alcohol-based skin disinfectants may have the appearance of ointments with creamy consistency, as those disclosed in the GB 2 017 49 1, or of a gel, for therapeutic or cosmetic use, in accordance with the GB 1 023 895.

The inventor has discovered a method of decreasing evaporation of alcohol-based skin disinfectants comprising adding alcohol-soluble viscosifying agents to these disinfectants, thereby increasing the exposure time and alcohol concentration of the disinfectant on the skin.

Alcohol solutions cointaining 30-900%, by weight, alcohol are often used by healthcare personnel to disinfect hands and for localized skin disinfection at the site of an invasive medical procedure. These alcohol compositions are very drying to the skin, evaporate quickly, and are easily spilled, due to their watery nature. These shortcomings are overcome by the instantly claimed method and compositions.

Description of the Invention

The instant invention is directed to a method for enhancing the efficacy of an alcohol-based skin disinfectant containing 30 to 90 percent, based on total composition weight, of at least one alcohol selected from the group consisting of n-propyl alcohol, isopropyl alcohol, and ethyl alcohol comprising adding 0,25 to 3 percent, based on total composition weight, of at least one alcohol-soluble hydroxypropyl cellulose polymer viscosifying agent, to said alcohol-based skin disinfectant, thereby retarding evaporation of said alcohol-based disinfectant and increasing its antimicrobial effectiveness.

The instant invention is also directed to an improved alcohol-based skin disinfectant composition comprising:

(a) 30 to to 90 percent, based on total composition weight of an alcohol selected from the group consisting of n-propyl alcohol, isopropyl alcohol and ethyl alcohol;

(b) 0,25 to 3 percent, based on total composition weight, an alcohol-soluble hydroxypropyl cellulose polymer viscosifying agent; and

(c) the balance water.

The present invention employs a viscosifying agent to retard alcohol evaporation, thereby allowing more efficient use of alcohols as skin disinfectants or antiseptics.

As disclosed previously, the preferred viscosifying agents are selected from the group consisting of hydroxypropyl cellulose polymers. While the molecular weight of the viscosifying agent is not critical, these preferred viscosifying agents are polymers with molecular weights sufficient to produce composition viscosities of at least 40 centipoise, when added to alcohol-based antimicrobial compositions at recommended dosages. More preferably, the viscosifiers produce viscosities of about 50 to about 500 cps in the final compositions.

As hydroxypropyl cellulose polymers, there may be mentioned Klucel HF and Klucel EF, which are hydroxypropyl cellulose ethers having molecular weights of about 1,000,000 and 60,000, respectively. These products are commercially available from Hercules, Incorporated. Hydroxypropyl cellulose polymers substantially reduce evaporation loss, thereby improving antibacterial efficacy.

Alcohol-based skin disinfectants and antiseptics are well-known in the art. As used herein, the terms "disinfectant" and "antiseptic" refer to those mixtures which are applied to skin for the purpose of killing bacteria and microorganisms on the skin. Such mixtures may be used as surgical scrub hand washes, patient pre-operative preparations, and as general healthcare hand washes. Other uses will become apparent to those skilled in the art, and are intended to be within the scope of this invention.

The preferred alcohol for use in the alcohol-based compositions of this invention is isopropyl alcohol.

Additionally, these compositions may contain fragrances, coloring agents, detackifiers such as cetyl lactate and additional antimicrobials without departing from the spirit of this invention.

As examples of preferred compositions, the following may be listed. These examples are not intended to limit the scope of the invention.

Formulation 1:

2

30-75 g n-propyl alcohol;
0.25-5.0 g hydroxypropyl cellulose;
color and fragrance as desired;
10-60 g deionized or distilled water.

Formulation 2:

30-75 g isopropyl alcohol;
0.25-5.0 g hydroxypropyl cellulose;
color and fragrance as desired;
10-60 ml deionized or distilled water.

Formulation 3:

40-80 g 95% ethyl alcohol;
0.25-5.0 g hydroxypropyl cellulose;
color and fragrance as desired;
5-50 ml deionized or distilled water.

Formulation 4:

30-75 g 2-isopropyl alcohol;
0.25-5.0 g hydroxypropyl cellulose;
0.5-2.0 g polymer of dimethyldiallyl ammonium
chloride (Merquat 100, used as an emollient,
available from Calgon Corporation,
Pittsburgh, PA);
0.1-0.2% para-chloro-meta-xylenol
color and fragrance as desired;
10-60 ml deionized or distilled water.

Formulation 5:

30-75 g isopropyl alcohol;
0.25-5.0 g hydroxypropyl cellulose;
0.5-2.0 g octyl palmitate (emollient);
0.25-0.5 g polymer of dimethyldiallyl
ammonium chloride;
0.1-0.5% chlorhexidine gluconate *;
color and fragrance as desired;
10-60 ml deionized or distilled water.

Formulation 6:

30-75 g isopropyl alcohol;
0.25-5.0 g hydroxypropyl cellulose;
0.5-2.0 g cetyl lactate;
0.25-0.5 g polymer of dimethyldiallyl
amnonium chloride;
color and fragrance as desired;

*para-chloro-meta-xylenol and chlorhexidine gluconate leave a residual antimicrobial agent on the skin after the alcohol has evaporated.

3

10-60 ml deionized or distilled water.

Examples 1 - 28

The following examples demonstrate the retardation of alcohol evaporation using viscosifying agents. Additionally, these examples demonstrate the antimicrobial efficacy of evaporation-stabilized, alcohol-based disinfectant/antiseptic compositions using pig skins as test substrates.

Preparation of Pig Skin:

Mature adult pig hides from freshly killed pigs were obtained from a slaughterhouse. The hides were washed using cold water and dehaired using a large animal clipper. After dehairing, the hides were cut into smaller sections with a scalpel, rinsed in cold water, placed in plastic bags, sealed and frozen.

Prior to use, each pigskin was tested for the presence of residual antibiotics. The method used was to randomly cut plugs, using a #7 cork borer, from the hide being tested and place them skin side down onto individual agar plates seeded with the test organisms. If a zone of inhibition surrounding the plug developed, the skin contained residual antibiotics and was not used.

Before use in a test, a section of pig skin was thawed, destubbled using a disposable razor, and defatted using a scalpel. The skin section was then rinsed in cold tap water and cut into 3 cm x 3 cm pieces using a scalpel. The 3 cm x 3 cm pieces were glued onto individual mounting holders (plastic caps approximately 4 to 5 cm in diameter or other suitable holders) with epoxy adhesive such that the skin surface was exposed. Two pieces of mounted skin were used for each sample tested. The mounted skins were placed into 100x20 mm petri dishes which contained a 7.0 cm filter paper disc moistened with approximately 1 ml of water to prevent drying. The prepared skins were placed in a refrigerator overnight.

Test Conditions:

The specific conditions used for the pig skin tests were as follows:

less than or equal to 10 organisms per cc
mixed inoculum consisting of Serratia
marcescens (ATCC 990), Escherichia coli (ATCC 8739), Staphylococcus aureus (ATCC 6538), Pseudomonas aeruginosa (ATCC 9027) and Candida albicans (ATCC 10231);
less than or equal to 2 hour incubation time between inoculation and treatment;
0.1 ml test sample;
15 second treatment (rubbing); and
3 minute air dry before imprinting.

Application of Test Organisms:

Suspensions of the various organisms were made by overlaying an overnight agar slant culture with 10 mls of Butterfield Buffer and gently rubbing the agar surface with a sterile pipet. These suspensions were mixed together to give a mixed inoculum of approximately $10^9$ microorganisms per ml. This mixed inoculum was further diluted to give either $10^7$ or $10^5$ organisms per ml depending upon the test conditions desired. One of two pieces of skin was inoculated with 0.1 ml of the diluted inoculum. The two pieces of skin were then rubbed together for 15 seconds and incubated at 30°C for either 15 minutes or up to 2 hours depending on test conditions desired.

Application of Alcoholic Preparations:

Three alcohols, n-propyl alcohol, isopropyl alcohol and ethyl alcohol, were tested. One of two pieces of inoculated skin was treated with 0.1 ml of the desired alcohol preparation. The two pieces of skin were then rubbed together for 15 seconds and allowed to air dry for 3 minutes to simulate normal evaporation before imprinting the skin samples onto the surface of a neutralizing growth media (Standard Methods Agar with Lecithin and Polysorbate 80 from BBL).

Imprints were made by inverting the mounting holder and pressing the treated skin onto the agar surface. Imprints were made after 3, 10 and 30 minutes and 1 hour. The plates were graded to indicate the extent of organism growth in the imprint. All of the samples were coded and graded blind to eliminate operator bias.

Ratings of 0 to 10 were given to denote coverage; i.e. 0 = no visible growth, 1 = 10% of the imprint surface covered, 5 = 50% of the imprint surface covered, etc.

Evaporation Rate Determination:

A measured volume of test solution was added near the center of two thicknesses of 12.5 cm filter paper in a large 150 mm petri dish. The dish was placed on a top loading balance sensitive to 0.01 g which was shielded on three sides and top against drafts and, as extra precaution, the air conditioner was turned off during the test period.

Weight readings were made each 30 seconds over a six-minute period demonstrating uniformity of loss over this period. Values reported are the average loss/minute over six minutes in mg/min. Each value reported represents a separate observation series.

Results are shown in Table I, below.

## TABLE I

### Evaporation Rate -- Pigskin Imprint Biological Comparison

| Example No. | Alcohol Viscosifier 4 ml Sample (% b/v) | (% w/w) | Average Evaporation Loss mg/min Run 1 | Run 2 | Coverage Rating** Imprint 3 min. | Imprint 10 min. | Imprint 30 min. | Imprint 1 hour |
|---|---|---|---|---|---|---|---|---|
| 1 | 40% IPA | 0% Klucel | 6.67 | 7.80 | | | | |
| 2 | 40% IPA | 0.25% Klucel | 4.17 | 4.17 | | | | |
| 3 | 40% IPA | 0.5% Klucel | 2.67 | 3.00 | | | | |
| 4 | 60% IPA | 0% Klucel | 7.5* | 5.67* | 10 | 10 | 10 | 10 |
| 5 | 60% IPA | 0.25% Klucel | 3.50 | 3.00 | 10 | 9.5 | 10 | 9.5 |
| 6 | 60% IPA | 0.5% Klucel | 3.16 | 2.83 | 9.5 | 10 | 9.5 | 9 |
| 7 | 60% IPA | 1.0% Klucel | 2.0 | 1.67 | 3 | 4 | 4 | 4 |
| 8 | 60% IPA | 3.0% Klucel | -- | -- | 3 | 3 | 3 | 3 |
| 9 | 80% IPA | 0% Klucel | 8.33* 7.50 | 4.33* 7.17 | 1.5 | 2 | 2 | 2 |
| 10 | 80% IPA | 0.25% Klucel | 4.00 | 3.33 | | | | |
| 11 | 80% IPA | 0.5% Klucel | 3.00 | 4.16 | | | | |
| 12 | 80% IPA | 0.5% Klucel | -- | -- | 1 | 1.5 | 1.5 | 1 |
| 13 | 60% n-Propanol | 0.0% Klucel | -- | -- | -- | 5 | 5 | 5 |
| 14 | 60% n-Propanol | 0.5% Klucel | -- | -- | -- | 2 | 2 | 2 |
| 15 | 60% n-Propanol | 0.75% Klucel | -- | -- | -- | 2 | 2 | 2 |

Table I (Continued):

| Example No. | Alcohol Viscosifier 4 ml Sample | | Average Evaporation Loss mg/min | | Coverage Rating** | | | |
|---|---|---|---|---|---|---|---|---|
| | (% b/v) | (% w/w) | Run 1 | Run 2 | Imprint 3 min. | Imprint 10 min. | Imprint 30 min. | Imprint 1 hour |
| 16 | 60% IPA | 0.5% Klucel and 0.2% UCAR | -- | -- | -- | 6.5 | 5.5 | 5.0 |
| 17 | 60% IPA | 0.5% Klucel and 0.5% UCAR | -- | -- | -- | 3.0 | 3.5 | 3.5 |
| 18 | 60% IPA | 0.5% Klucel and 0.5% UCAR | -- | -- | -- | 2.5 | 2.5 | 2.5 |
| 19 | 40% IPA | 0% Klucel | -- | -- | 10 | 10 | 10 | 10 |
| 20 | 40% IPA | 0.5% Klucel | -- | -- | 8 | 8.5 | 8.5 | 8 |
| 21 | 40% IPA | 1.0% Klucel | -- | -- | 9.5 | 9.5 | 9.5 | 9.5 |
| 22 | 40% IPA | 2.0% Klucel | -- | -- | 8 | 8 | 7.5 | 7.5 |
| 23 | 60% Ethanol | 0% Klucel | 5.67 | 8.17 | 7.5 | 7 | 6 | 5 |
| 24 | 60% Ethanol | 0.5% Klucel | 1.67 1.33 | 2.5 -- | 6 | 6 | 5 | 4 |

Use 2 ml Sample:

| Example No. | (% b/v) | (% w/w) | Run 1 | Run 2 |
|---|---|---|---|---|
| 25 | 60% IPA | 0% Klucel | 4.83 | 6.67 |
| 26 | 60% IPA | 0.5% Klucel | 2.17 | 2.00 |

EP 0 223 681 B1

Table I (Continued):

| Example No. | Alcohol Viscosifier 4 ml Sample (% b/v) | (% w/w) | Average Evaporation Loss mg/min | | Coverage Rating** | | | |
|---|---|---|---|---|---|---|---|---|
| | | | Run 1 | Run 2 | Imprint 3 min. | Imprint 10 min. | Imprint 30 min. | Imprint 1 hour |
| Use 6 ml Sample: | | | | | | | | |
| 27 | 60% IPA | 0% Klucel | 8.83 | 8.67 | | | | |
| 28 | 60% IPA | 0.5% Klucel | 3.83 | 3.67 | | | | |

*Average of two tests.
**Coverage Rating: 0 = No growth   10 = 100% coverage   5 = 50% coverage.

Klucel = Klucel HF (hydroxypropyl cellulose), unless otherwise indicated as Klucel EF, available from Hercules, Inc.

UCAR = Ucare Polymer JR-400 (quaternary ammonium salt of hydroxyethyl cellulose reacted with trimethylammonium substituted epoxides), available from Union Carbide Corporation.

These examples demonstrate evaporation retardation and the improved antibacterial activity of the instant invention.

**Claims**

1. A method for enhancing the efficacy of an alcohol-based skin disinfectant containing 30 to 90 percent, based on total composition weight, of at least one alcohol selected from the group consisting of n-propyl alcohol, isopropyl alcohol, and ethyl alcohol comprising adding 0,25 to 3 percent, based on total composition weight, of at least one alcohol-soluble hydroxypropyl cellulose polymer viscosifying agent, to said alcohol-based skin disinfectant, thereby retarding evaporation of said alcohol-based disinfectant and increasing its antimicrobial effectiveness.

2. The method of Claim 1, wherein said alcohol-base skin disinfectant contains isopropyl alcohol.

3. An improved alcohol-based skin disinfectant composition comprising:
   (a) 30 to to 90 percent, based on total composition weight of an alcohol selected from the group consisting of n-propyl alcohol, isopropyl alcohol and ethyl alcohol;
   (b) 0.25 to 3 percent, based on total composition weight, an alcohol-soluble hydroxypropyl cellulose polymer viscosifying agent; and
   (c) the balance water.

4. The composition of Claim 3, wherein said alcohol is isopropyl alcohol.

5. The composition of Claim 3, wherein said alcohol is 50 to 70 percent of said composition, based on total composition weight.

## Revendications

1. procédé pour rehausser l 'efficacité d'un désinfectant dermique à base d'alcool contenant de 30 à 90% par rapport au poids total de la composition d'au moins un alcool choisi parmi l 'alcool n-propylique, l 'alcool isopropylique et l 'alcool éthylique, qui consiste à ajouter de 0,25 à 3% par rapport au poids total de la composition d'au moins un agent de viscosité qui est un polymère d' hydroxypropylcellulose soluble dans l 'alcool audit désinfectant dermique à base d'alcool pour ainsi retarder l' évaporation dudit désinfectant à base d'alcool et en augmenter l'efficacité antimicrobienne.

2. Procédé selon la revendication 1, dans lequel ledit désinfectant dermique à base d'alcool contient de l'alcool isopropylique.

3. Composition désinfectante améliorée de la peau a base d'alcool qui comprend :
   a) de 30 à 90% par rapport au poids total de la composition d'un alcool choisi parmi l 'alcool N-propylique, l'alcool isopropylique et l'alcool éthylique;
   b) de 0,25 à 3% du poids total de la composition d'un agent de viscosité qui est un polymère d'hydroxypropylcellulose soluble dans l'alcool ; et
   c) le complément étant de l'eau.

4. Composition selon la revendication 3, dans la-quelle ledit alcool est l'alcool isopropylique.

5. Composition selon la revendication 3, dans la-quelle ledit alcool représente de 50 à 70% de ladite composition par rapport au poids total de la composition.

## Ansprüche

1. Verfahren zur Erhöhung der Wirksamkeit eines Hautdesinfektionsmittels auf Alkoholbasis mit 30 bis 90 %, bezogen auf das Gesamtgewicht der Zusammensetzung, wenigstens eines Alkohols, ausgewählt aus der aus n-Propylalkohol, Isopropylalkohol und Ethylalkohol bestehenden Gruppe, durch Zugabe von 0,25 bis 3 %, bezogen auf das Gesamtgewicht der Zusammensetzung, wenigstens eines alkohollöslichen Hydroxypropylcellulosepolymers als die Viskosität erhöhendes Mittel zu dem Hautdesinfektionsmittel auf Alkoholbasis, wodurch die Verdampfung des Desinfektionsmittels auf Alkoholbasis verzögert und seine antimikrobielle Wirksamkeit erhöht wird.

2. Verfahren nach Anspruch 1, worin das Hautdesinfektionsmittel auf Alkoholbasis Isopropylalkohol enthält.

3. Verbesserte Hautdesinfektionsmittelzusammensetzung auf Alkoholbasis, welche umfaßt:

(a) 30 bis 90 %, bezogen auf das Gesamtgewicht der Zusammensetzung, eines Alkohols, ausgewählt aus der aus n-Propylalkohol, Isopropylalkohol und Ethylalkohol bestehenden Gruppe;
(b) 0,25 bis 3 %, bezogen auf das Gesamtgewicht der Zusammensetzung, eines alkohollöslichen Hydroxypropylcellulosepolymers als die Viskosität erhöhendes Mittel; und
(c) als Rest Wasser.

4. Zusammensetzung nach Anspruch 3, worin der Alkohol Isopropylalkohol ist.

5. Zusammensetzung nach Anspruch 3, worin der Alkohol 50-70 % der Zusammensetzung, bezogen auf das Gesamtgewicht der Zusammensetzung, ausmacht.